(19) Europäisches Patentamt
European Patent Office
Office européen des brevets

(11) **EP 0 441 917 B1**

(12) **EUROPEAN PATENT SPECIFICATION**

(45) Date of publication and mention
of the grant of the patent:
**01.05.1996 Bulletin 1996/18**

(21) Application number: **90911551.1**

(22) Date of filing: **12.07.1990**

(51) Int. Cl.[6]: **A61K 47/48**, A61K 39/395,
C12N 5/10

(86) International application number: **PCT/US90/03921**

(87) International publication number:
**WO 91/01145 (07.02.1991 Gazette 1991/04)**

(54) **POKEWEED ANTIVIRAL PROTEIN - MONOCLONAL ANTIBODY CONJUGATES**

KONJUGATE VON "POKEWEED" ANTIVIRALEM PROTEIN UND MONOKLONALEN ANTIKÖRPERN

CONJUGUES DE PROTEINES ANTIVIRALES POKEWEED - ANTICORPS MONOCLONAUX

(84) Designated Contracting States:
**AT BE CH DE DK ES FR GB IT LI LU NL SE**

(30) Priority: **25.07.1989 US 385314**
**30.03.1990 US 503522**

(43) Date of publication of application:
**21.08.1991 Bulletin 1991/34**

(73) Proprietors:
• **THE REGENTS OF THE UNIVERSITY OF MINNESOTA**
**Minneapolis, Minnesota 55455 (US)**
• **ONCOGEN LIMITED PARTNERSHIP**
**Seattle, WA 98121 (US)**

(72) Inventors:
• **UCKUN, Fatih, M.**
**Minneapolis, MN 55418 (US)**
• **ZARLING, Joyce, Mintzer**
**Seattle, WA 98199 (US)**

(74) Representative: **Eisenführ, Speiser & Partner**
**Martinistrasse 24**
**D-28195 Bremen (DE)**

(56) References cited:
• **Meeting on Human Retroviruses held at the 18th Annual Meeting of the UCLA (University of California, Los Angeles) Symposia on Molecular and Cellular Biology, Tamarron, Colorado, 4-11 February 1989, F.M. UCKUN et al.: "Pokeweed Antiviral Protein-Monoclonal Antibody Conjugates Selectively Inhibit HIV-1 Replication in Human T-Cells and Monocytes", see Abstract G 448, & J. Cell Biochem. Suppl. 0 (13 part B), 1989**
• **The Journal of Immunology, Vol. 124, No. 6, June 1980, The Williams & Wilkins Co, (Baltimore, US), J.P. VAN WAUWE et al.: "Okt3: a Monoclonal Anti-Human T Lymphocyte Antibody with Potent Mitogenic Properties", pages 2708-2713, see Abstract**
• **J.Cell. Biochem. Suppl.0(13 part B),1989 (abstract)**

**Description**

The present invention relates to an antiviral composition as defined in the preamble of claim 1; it further relates to a method for inhibiting HIV replication in mammalian cells.

The acquired immunodeficiency syndrome (AIDS) and infection with the human immunodeficiency virus type 1 (HIV-1) constitute a worldwide public health problem. (A. Venkatesan, Science 241: 1481-1485 (1988). At present, there is no cure for AIDS, and available treatment modalities to reduce HIV production in vivo such as the use of AZT cause toxic side effects.

HIV is an RNA retrovirus that was originally designated human T lymphotropic virus (HTLV)-III, lymphadenopathy-associated virus (LAV), or AIDS associated retrovirus (ARV). Fauci, Science 239: 617-622 (1988). The virus shares many features with other members of the nontransforming and cytopathic lentivirus family of retroviruses. HIV is referred to as HIV-1 to differentiate it from HIV-2 which has been isolated from West African patients with a clinical syndrome indistinguishable from HIV-induced AIDS. The critical basis for the immunopathogenesis of HIV infection is the depletion of the CD4+ helper/inducer subset of T-cells, resulting in profound immunosuppression. See Dahlgleish et al, Nature 312: 763-767 (1984); Fauci, Clin. Res. 32: 491-496 (1985), Ho et al. N. Engl. J. Med. 317: 278-281 (1987). HIV has a selective tropism for CD4+ T-cells and macrophages, and the CD4 antigen is an essential component of the cell surface receptor for HIV-1, HIV-2, as well as SIV. Lasky et al. Science 233: 209-212 (1986); Lasky et al, Cell 50: 977-985 (1987). After HIV binds to the CD4 molecule via the external envelope glycoprotein gp 120, the virus is internalized and uncoated. Fauci, Science 239: 617-622 (1988); Stein et al. Cell 49: 659-669 (1987). Once internalized, the genomic RNA is transcribed to DNA by the enzyme reverse transcriptase. The proviral DNA is then integrated into the host chromosomal DNA. After integration of the provirus, the infection may assume a latent phase or the proviral DNA may transcribe viral genomic RNA and messenger RNA. Protein synthesis, processing, and virus assembly occur with budding of the mature viron from the cell surface. At present, there is no cure for AIDS, and AZT, the only approved antiviral drug to reduce HIV production in vivo, can cause major toxic side effects and can result in the emergence of AZT resistant mutants. Long term treatment for HIV infections may require multiple drugs given sequentially or in combination similar to combination chemotherapy required for many cancers.

Antibody conjugates composed of hybridized or conjugated monoclonal antibody and toxin have been used to eradicate specific populations of target cells by homing in and destroying "unwanted" target cells bearing the target surface antigens. See, e.g. Jansen et al., Immunol. Rev., 62: 185 (1982), Leonard et al., Cancer Research 45: 5263 (1985); Uckun, Journal of Experimental Medicine, 163, 347-368 (1986), Uckun et al., Cancer Research, 45, 69-75 (1985); Ramakrishnan et al., Journal of Immunology, 135, 3516-3522 (1985). The variety of toxins that have been employed by various investigators can be broadly categorized into two groups. The first group consists of intact toxins, such as intact ricin. See, e.g. Leonard et al., supra. These toxins can not be safely applied in vivo because of lethal toxicity. The second group of toxins are referred to as hemitoxins. Hemitoxins are single-chain ribosome inactivating proteins that act catalytically on eukaryotic ribosomes and inactivate the 60-S subunit, resulting in a dose dependent inhibition of cellular protein synthesis at the level of peptide elongation. See Barbieri & Stirpe, Cancer Surveys, 1: 489 (1982).

One hemotoxin of interest is pokeweed antiviral protein (PAP) which is isolated from spring leaves of late summer leaves and seeds of Phytolacca americana. PAP has been recognized as having antiviral activity for many years. See Aron & Irvin, Antimicrob. Agents Chemother. 17: 1032 (1980); Barbieri et al, supra. PAP has been shown to block the transmission of RNA-containing viruses in plants such as tobacco mosaic virus, [Irvin, Arch. Biochem. Biophys 200: 418 (1980)] and cucumber mosaic virus. [Tomlison et al., J. Gen. Virol., 22: 225 (1974)]. PAP has also been reported to inhibit the replication of two RNA - containing animal viruses: poliovirus [Ussery et al., Ann. N.Y. Acad. Sci., 284; 431-440 (1977)] and influenza virus. Tomlinson et al., supra. PAP has also been reported to inhibit multiplication of herpes simplex virus type I (Aron, supra) and type II (United States Patent No. 4,672,053 to Obrig.) Although PAP-conjugates of G3.7/CD7, F13/CD14 and B43/CD19 monoclonal antibodies have been reported to inhibit HIV-1 replication, these conjugates have been inconsistent in their ability to inhibit virus replication (Uckun et al., Mtg. Human Retrov., 18th Ann. Mtg. UCLA Symp. Moles. Cell. Biol., Feb. 4-11, 1989, J. Cell Biochem. Suppl. O (13 Part B) (1989)).

Chemical agents have been used to stimulate mitosis in human peripheral T lymphocytes. Such mitogenic agents include sodium periodate, phorbol myristate acetate, and galactose oxidase. T cell phytomitogens are considered to initiate lymphocyte mitosis by binding with specific sites on the lymphocyte membrane. OKT3, an anti-human T cell antibody that binds to an unresolved T cell surface determinant, has also been described as being mitogenic for human peripheral mononuclear cells. (van Wauwe et al., J. Immunol. 124:2708-2713 (1980)).

Hemitoxin-containing antibody conjugates have highly specific site-directed cytotoxicity resulting from binding target cells solely via the specific monoclonal antibody moiety of the conjugate. Antibody conjugates containing PAP have been studied and shown to be cytotoxic for malignant cells at sufficiently high concentrations by inhibiting protein synthesis. Covalent binding of PAP to an immunoglobulin which is capable of binding selectively to an antigen specific to a cell to be killed was described in United States Patent No. 4,363,758 to Masuho. However, binding of PAP to a monoclonal antibody directed against virus infected cells to inhibit viral replication without cytotoxicity has not been described or discussed before. In fact, steady concentrations of PAP shown to have antiviral activity can not be achieved in vivo since: 1) they would be toxic; and 2) the half life of PAP in vivo is about 5-10 minutes.

It is the object of the invention to provide antibody conjugates composed of monoclonal antibodies conjugated to pokeweed antiviral protein which can target PAP to inhibit virus replication in infected cells without resulting in death of uninfected cells.

This object is attained by an antiviral composition as defined in claim 1 and by a method as defined in claims 4 and 7. Preferred embodiments are defined in the sub-claims.

The present invention is directed to antiviral-antibody conjugates composed of a $CD_4$ or $CD_5$ antigen monoclonal antibody and pokeweed antiviral protein (PAP) isolated from Phytolacca Americana. The present invention provides for monoclonal antibody-PAP antiviral compositions useful for inhibiting intracellular virus replication associated with diseases such as HIV-induced AIDS or retrovirus induced T-cell leukemias. The present invention employs monoclonal antibodies or fragments thereof reactive with an antigen present at the surface of virus infected mammalian cells. Monoclonal antibody conjugates of the present invention retain the ability of non-conjugated PAP to inhibit cellular protein synthesis.

We have discovered that monoclonal antibody-PAP conjugates can effectively inhibit virus replication without detectable cytotoxicity. Using the present invention, in the case of HIV, monoclonal antibody-PAP conjugates in amounts less than about 35 pico molar (pM) can inhibit HIV replication by at least 50% without detectable cytotoxicity.

Monoclonal antibody - PAP conjugates of the present invention inhibit HIV replication in CD4+ T-cells and monocytes by at least 50% at levels about 25 times less than levels of PAP required to inhibit viral replication by 50%. Preferably, monoclonal antibody-PAP conjugates of the present invention inhibit HIV replication in CD4+ cells and monocytes by about 50% at levels about 100 times less than that required in the case of non-conjugated PAP (and more preferably 200 times less than required in the case of non-conjugated PAP). In one embodiment of the invention, PAP conjugate of a monoclonal antibody to monocyte cell surface antigen CD14 effectively abrogates HIV production in human monocytes at a concentration of less than about 150 pM.

The antibody-PAP conjugates of the present invention are PAP conjugates of a monoclonal antibody to T-cell surface antigen CD4 and a monoclonal antibody to T-cell surface antigen CD5. Also, HIV replication in human CD4+ T-cells can be inhibited at least 50% by employing PAP conjugates of monoclonal antibodies to CD4 or to CD5 T-cell antigens at concentrations below about 35 pM without inhibiting cell replication. Furthermore, production of HIV-1 in activated CD4+ T-cells from HIV-1 infected patients can be inhibited by at least 50% by employing PAP conjugated to a monoclonal antibody to CD4 at concentrations below about 10 pM without inhibiting proliferation of CD4+ T-cells.

The present invention also provides a method for inactivating or inhibiting replication of HIV in mammalian cells which involves treating a cell culture including HIV infected cells with a monoclonal antibody-PAP conjugate composition. Using the method of the present invention HIV replication can be effectively inhibited by at least 50% without inhibiting cell replication. Further, the PAP-monoclonal antibody conjugates of the present invention are not toxic to normal bone marrow progenitor cells CFU-GM (myeloid progenitor cell=colony forming unit-granulocyte-macrophage), BFU-E (erythroid progenitor cell=burst forming unit-erythroid), or CFU-GEMM (pluripotent progenitor cell=colony forming unit-granulocyte-erythroid-macrophage-megakaryocyte) at 5-15 nM concentrations which are much higher than those to inhibit HIV replication by 50%. PAP-monoclonal antibody conjugates of the present invention can inhibit virus replication by at least 50% at levels at least 25 times less and preferably by at least 50% at levels at least 100 times less than levels of the conjugate required to inhibit 50% of cell replication. Further, PAP-monoclonal antibody conjugates can inhibit substantially all virus replication at a concentration of conjugate that inhibits cell replication by less than 50%.

It is expected that monoclonal antibody-PAP conjugates will provide the basis for a highly effective procedure to inhibit HIV replication in mammalian monocytes and T-cells; thereby, providing a method to treat patients with AIDS or patients infected with HIV-1 who have not yet developed AIDS. It is further expected that monoclonal antibody-PAP conjugates will provide the basis for an effective method to inhibit other retroviruses (HTLV-1, etc.) and viruses other than retroviruses such as, but not limited to, members of the herpes virus group (HSV, CMV, EBV), influenza viruses, rhinoviruses, papovaviruses (human papilloma), adenoviruses, hepatitis virus, etc.

Figure 1 illustrates PAP monoclonal antibody conjugation procedure;

Figure 2 shows how PAP monoclonal antibody conjugate is purified;

Figures 3A and 3B demonstrate two representative examples for HPLC profiles of: (a) PAP-mAb conjugates and (b) purified PAP-mAb conjugates;

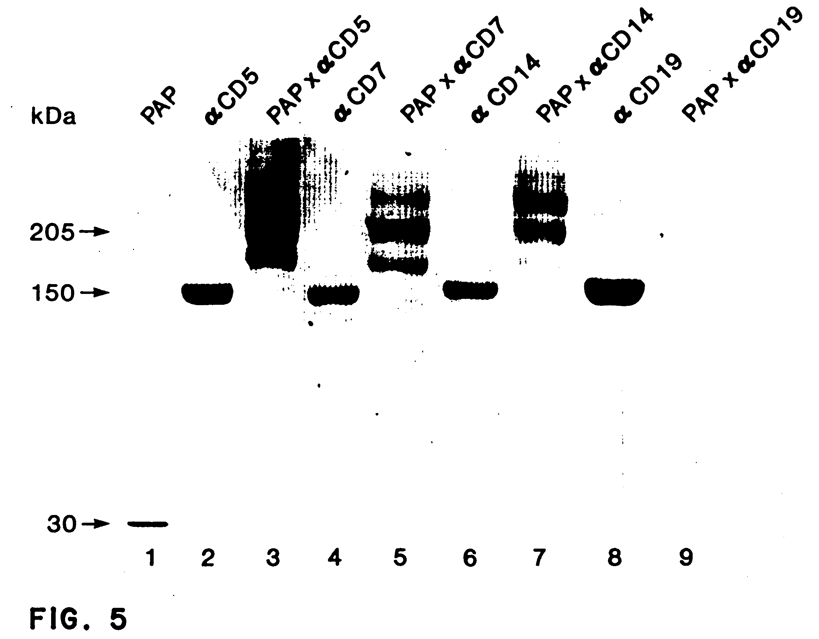

Figures 4 and 5 show the SDS-PAGE analysis of monoclonal antibodies and PAP-mAb conjugates. SDS PAGE of CD14 antigen directed F13 mAb, and F13-PAP conjugate (before and after purification), CD7 antigen directed G3.7-mAb, G3.7-PAP mAb conjugate (before and after purification), and CD5 antigen directed T101 mAb, T101-PAP conjugate (after purification) and CD4 antigen directed G17-2 mAb, G17-2 mAb conjugate (after purification) on a 5% SDS gel. The gels were stained with Coomassie Blue and the positions of the molecular weight markers are indicated on the left.

Figure 6 shows the effect of non-conjugated PAP on HIV replication (p24 production), in human CD4$^+$ T-cells and proliferation of these cells ($^3$H-TdR incorporation).

Figure 7 shows the effect of PAP-anti-CD5 (T101) conjugate on HIV replication (p24 production), in human CD4$^+$ T-cells and proliferation of these cells ($^3$H-TdR incorporation).

Figure 8 shows the effect of PAP-anti-CD7 (G3.7) conjugate on HIV replication (p24 production), in human CD4$^+$ T-cells and proliferation of these cells ($^3$H-TdR incorporation).

Figure 9 shows the effect of PAP-anti-CD19 (B43) on HIV replication (p24 production) in human CD4$^+$ T-cells that do not react with anti-CD 19.

Figure 10 shows the effect of PAP-anti-CD4 (G17-2) conjugate on HIV replication (p24 production), in human CD4$^+$ T-cells and proliferation of these cells ($^3$H-TdR incorporation).

Figures 11A and 11B show the effect of PAP-anti-CD4 (G17-2) conjugate on HIV replication (p24 production) in peripheral blood lymphocytes (PBL) from two HIV-1 infected patients, that were activated with mAb to CD3 to induce HIV-1 production, and the effects of PAP-anti-CD4 on proliferation of these cells ($^3$H-TdR incorporation).

Figures 12A and 12B show the effect of continuous 22-day treatment and 5-day treatment with PAP-anti-CD4 on HIV-1 production (p24 production) for 22 days in PBL from two HIV-1 infected patients after stimulating the PBL with mAb to CD3 to induce HIV-1 production.

Figure 13 shows a composite graph of anti-HIV activity in CD4$^+$ cells by PAP, PAP-anti-CD5 and PAP-anti-CD7.

Figure 14 shows that (a) the PAP-mAb conjugates are cytotoxic to the target cells at much higher concentrations than those which inhibit HIV-1 replication and (b) they are not toxic to normal bone marrow stem cells (CFU-GM, BFU-E, CFU-GEMM) up to concentrations of 5 x 10$^3$ pM.

The present invention is directed to PAP-mAb conjugates useful to inhibit intracellular replication of mammalian viruses, including in a preferred embodiment retroviruses such as HTLV-1, HTLV-11, SIV, HIV-1 and HIV-2 or the like in mammalian cells. The PAP-mAb conjugates of the present invention are most specifically directed to inhibiting HIV replication in human monocytes and T-cells.

The present invention provides antiviral-antibody conjugates which are capable of inhibiting virus replication in mammalian cells without detectable cytotoxicity. For purposes of the present invention detectable cytotoxicity refers to levels of cell growth inhibition measurable or observed by $^3$H-Leucine incorporation assays, clonogenic assays or $^3$H-Thymidine ($^3$H-TdR) incorporation. As described herein antiviral-antibody conjugates refer in general to a monoclonal antibody (mAb) and antiviral protein conjugate wherein the antibody is covalently bonded or cross-linked to the antiviral protein. More specifically, the antibody conjugates of the present invention include monoclonal antibodies or antigen-binding fragments thereof reactive with surface active antigens on target cells. The monoclonal antibody or its antigen binding fragment is covalently linked to pokeweed antiviral protein (PAP); a purified protein extracted from Phytolacca Americana capable of inhibiting protein synthesis.

By using PAP-mAb conjugates of the present invention at least 50% of virus replication can be inhibited without inhibiting cell replication. PAP-mAb conjugates of the present invention can inhibit virus replication by at least 50% using PAP-mAb conjugate in concentrations below about 150 pM without inhibiting cell replication. We have observed about 50% inhibition of HIV replication in human CD4$^+$ T-cells without inhibiting cell replication at concentrations less than 35 pM using PAP conjugated to mAb against CD5 or CD4. In one embodiment approximately 200 times more non-conjugated PAP is required to cause 50% inhibition of HIV replication.

In the present invention, the PAP-mAb conjugate is reactive with an antigen at or on the surface of mammalian cells, such as monocytes, human T-cells, epithelial cells, brain cells and the like. As described herein "surface antigen" of virus infected cells refers to antigens associated with virus infected cells constituting, for example, normal differentiation antigens on those cells which are expressed regardless of virus infection as well as antigens expressed or associated by cells only when such cells become infected with a particular virus.

<u>PAP Source & Purification</u>

PAP is a protein capable of inhibiting protein synthesis obtained from Phytolacca Americana. The protein, has a molecular weight of about 30,000 and comprises a single polypeptide chain. PAP can be extracted from Phytolacca Americana and purified according to a publicly known method, such as the method described by J.D. Irvin, <u>Pharmac. Ther. 21:</u> 371-387 (1983); Uckun, <u>ANTIBODY, 1,</u> 247-262 (1988); PAP has a strong activity to inhibit the synthesis of polyphenylalanine with the use of ribosome of reticulocyte. PAP can be extracted not only from the leaves of Phytolacca Americana but also from its seeds.

<u>Monoclonal Antibodies</u>

The general techniques for producing monoclonal antibodies are based on the fusion of spleen lymphocytes with malignant cells (myelomas) of bone marrow primary tumors [C. Milstein, <u>Sci. Am.</u>, <u>243</u>, 66 (1980)]. The methods yield a hybrid cell line, arising from a single fused cell hybrid, or clone, which possesses characteristics of both the lymphocytes and myeloma cell lines. Like the lymphocytes (taken from animals primed with sheep red blood cells as antigens), the fused hybrids or hybridomas secrete antibodies (immunoglobulins) reactive with the antigen. Moreover, like the myeloma cell lines, the hybrid cell lines are immortal. Specifically, whereas antisera derived from vaccinated animals are variable mixtures of antibodies which cannot be identically reproduced, the single type of immunoglobulin secreted by a hybridoma is specific to one and only one antigenic determinant on the antigen, a complex molecule having a multiplicity of antigenic molecular substructures, or determinates (epitopes). For instance, if the antigen is a protein, an antigenic determinant may be one of the many peptide sequences within the entire protein molecule. Hence, monoclonal antibodies raised against a single antigen may be distinct from each other depending on the determinant that induced their formation. However, all of the antibodies produced by a given clone are identical. Furthermore, preferred hybridoma cell lines can be reproduced indefinitely, are easily propagated in vitro or in vivo, and yield monoclonal antibodies in extremely high concentrations. Monoclonal antibodies envisioned for use in the present invention can be of mouse origin, human antibodies or chimeric antibodies "half mouse" and "half human".

As indicated above, the present invention uses monoclonal antibodies reactive with an antigen present at the surface of virus infected mammalian cells such as monocytes and human T-cells, epithedial cells and the like. Monoclonal antibodies useful in the present invention are produced using well known hybridoma fusion techniques [G. Kohler and C. Milstein, Eur. J. Immunol., 6: 511 (1976); M. Shulman et al., Nature, 276: 269 (1978)]. Monoclonal antibodies that can be used in the present invention are those directed against primate cell surface antigens, namely CD4 and CD 5, wherein G17-2/CD4, T101/CD5, 10.2/CD5 and G19-4/CD3 are particularly preferred. Example antibodies - which are in part described for comparative purposes - include Mab F13 against CD14; G3.7 against CD7; T101 against CD5; and mAb G17-2 against CD4. All antibodies are described in Ledbetter et al., Molecular Immunology, 26, 137-145 (1989); Uckun et al., J. of Immunology, 140, 2103-2111 (1988); Blood, 66:627-635 (1985); Ledbetter et al., Molecular Immunology, 24:1255-1267 (1987); see also the literature references given under item I of the examples.

Preparation and Characteristics of Immunotoxins

(a) Specificity Testing

The binding reactivity of F13, G3.7, T101 and G17-2 and of the other monoclonal antibodies employed in the present description can be evaluated by the indirect immunofluorescence assay. In this assay, the cells to be tested are contacted in a suspension or on a slide with an excess of the mAb. After a suitable incubation period, the cells were washed to free them of unbound mAb and the bound antibody detected with an anti-mouse antibody bound to a fluorescent label such as fluorescein isothiocyanate (FITC).

The binding of the monoclonal antibody PAP conjugate to T-cells and monocytes can be determined by a "double sandwich" type immunofluorescence assay wherein the cells to be assayed are incubated sequentially with (a) the PAP -monoclonal antibody conjugate, (b) an antiserum to the pokeweed antiviral protein, and (c) a fluorescent-labelled antibody to the antiserum. The labelled cells can be analyzed by cytofluoremetic techniques using appropriate background substraction techniques.

(b) Evaluation of PAP-Monoclonal Antibody Conjugate Cytotoxicity

Cells can be contacted with the monoclonal antibody-PAP conjugate by suspending the cells in a suitable physiological medium and adding the antibody-PAP conjugate to the desired concentration. Following antibody-PAP conjugate treatment, the survival of the cells can be measured using: a) protein synthesis inhibition assays; b) trypan blue viability assays; c) clonogenic assays by limiting dilution as described by Uckun et al., Cancer Res.,45: 69-75 (1985); Uckun et al. Autologous Bone Marrow Transplant. Proc. First Intl. Symp., 449-453 (1985); Uckun et al. J. of Immunol. 134: 2010-2016 (1985); and d) $^3$H-thymidine incorporation to measure cellular DNA synthesis. Also, the effect on survival of the cells can be measured by suspending the cells in alpha-MEM or RPMI 11640 medium (Grand Ble. NY) which can be supplemented with phytohemagglutin-stimulated lymphocyte conditioned medium (PHA-LCM) or PHA with or without interleukin-2. Samples of the cell suspension are then cultured for two weeks and any viable cells are detected by microscopy using trypan blue dye exclusion assay described in Uckun et al. J. of Immunol., 134: 2010-2016, (1985) or the cells are labelled with $^3$H-thymidine as a measure of cell replication.

(c) Evaluation of PAP-Monoclonal Antibody Conjugates on Virus Replication

Uninfected cells such as monocytes and fresh CD4$^+$ T-cells are treated with monoclonal antibody-PAP conjugates. Samples of treated cells are then exposed to HIV. After about 2 hour absorption with the virus, cell free virus is removed and new medium containing monoclonal antibody-PAP conjugate is added. Samples of the cell suspension are cultured for five to eight days at which time supernate is removed and assayed for the presence of HIV p-24 activity in antigen capture ELISA assays. Alternatively, PBL from HIV-1 infected patients are treated with monoclonal antibody-PAP conjugates and activated with an agent effective to induce cell proliferation and HIV replication such as a monoclonal antibody to CD3 or a mitogen such as phytohemagglutin or the like. The amount of agent needed to activate HIV replication will vary from about 0.1 to 1.0 µg/ml depending on the type of agent. In the case of mAb to CD3, from about 0.5 to 2.0 µg/ml is required. As described herein, mAb to CD3 (G19-4) was used to activate the cells to replicate and produce HIV-1. Samples of the cell suspension are cultured for five days to several weeks at which time supernate is removed and assayed for the presence of HIV p-24 activity in antigen capture ELISA assays.

Therapeutic Use

Patient treatment using the composition of the present invention involves administering therapeutic amounts of the monoclonal antibody and PAP conjugate composition. In the context of the present invention, the terms "treat" and "therapy" and the like refer to prophylaxis or attenuation of existing disease. The antibody-PAP composition may be formulated with conventional pharmaceutically acceptable parenteral vehicles for administration by injection. These vehicles comprise substances which are essentially nontoxic and nontherapeutic such as water, saline, Ringer's solution, dextrose solution, Hank's solution or the like. It is to be understood that antibody-PAP conjugate formulations may also include small amounts of adjuvants such as buffers and preservatives to maintain isotonicity, physiological and pH stability. Preferably, the antibody-PAP conjugate is formulated in purified form substantially free of aggregates and other protein at concentrations ranging from about 0.1 to about 10mg/ml.

As indicated by the above formulation, the PAP - monoclonal antibody conjugates may be administered parenterally. In the case of some virus diseases, PAP-monoclonal antibody conjugates can be delivered or administered topically, intravenously, or in aerosol form. When PAP - monoclonal antibody conjugates are administered intravenously they can be delivered as a bolus or on a continuous basis.

The dose of the antibody-PAP conjugate formulation to be administered will depend upon the patient and the patient's medical history. However, the dose should be sufficient to inhibit a substantial portion, usually more than about 90%, of the virus replication in infected cells of the patient. For example: PAP-anti-CD7 or PAP-anti-CD4 would inhibit HIV-replication by >90% if used at 10-100 pM range, which is equal to 2.0-20 ng/ml. The dose required to achieve this concentration can be calculated using the formula: Dose in micrograms = 70 x 2(20) x wt (in kg)/1,000. For a 70 kg patient, this would yield 10-100 micrograms. Notably, these doses are 50-500 fold smaller than those tolerated by non-human primates with no detectable toxicity. Dosages for adult humans with HIV infection envisioned by the present invention and considered to be therapeutically effective will therefore range from between about 10 and 100 micrograms and will be administered with a frequency based on the plasma half life of PAP-mAb conjugates in a given patient, as determined by solid phase ELISA. Doses can readily be adjusted to provide appropriate amounts of the antibody-PAP conjugate to children using the above formula.

The invention will be further described by reference to the following detailed examples, wherein antibodies not relating to the claims (such as F13-PAP, G3.7-PAP or CD7, CD14 or CD19 conjugates) serve for comparative purposes.

EXAMPLES

I. Monoclonal Antibodies

The following monoclonal antibodies were employed in conjugates with PAP.

Monoclonal antibody F-13 described in Andrews et al. "Leucocyte Typing: Human Leucocyte Differentiation Antigens Detected by Monoclonal Antibodies," pp. 398-404 (edited by A. Bernard et al. 1984).

Monoclonal antibody G3.7 described in Uckun et al., J. of Immunology, 140, 2103-2111 (1985) and Uckun et al., J. of Immunology, 135, 3516-3522 (1985) and deposited with ATCC on July 20, 1989 under ATCC No. HB10182.

Monoclonal antibody T101 described in Royston et al., Transpl. Proc. 13, 761-766 (1981); Uckun et al., Blood, 69, 361-366 (1987).

Monoclonal antibody B43 described in Uckun et al., Immunology, 134, 2010-2016 (1985) (ATCC No. HB8903).

Monoclonal antibody G17-2 described in Ledbetter et al., Molecular Immunology, 24, 1255-1261 (1987) and deposited with ATCC on March 29, 1990 under ATCC No. HB10402.

II. Synthesis of PAP - Monoclonal Antibody Conjugates

Pokeweed antiviral protein (PAP) is a single chain polypeptide toxin (m.w. 30,000) that catalytically inactivates the 60S subunit of eukaryotic ribosomes and can be isolated from the spring leaves of pokeweed (Phytolacca Americana) as described by L.L. Houston et al., in J. Biol. Chem., 25 9601 (1983). PAP is purified as described by Irvin, Pharmac. Ther. 21: 371-383 (1983); Uckun, ANTIBODY, 1, 247-262 (1988) the disclosure of which is incorporated by reference herein; Uckun, J. of Immunology, 134, 2010-2016 (1985).

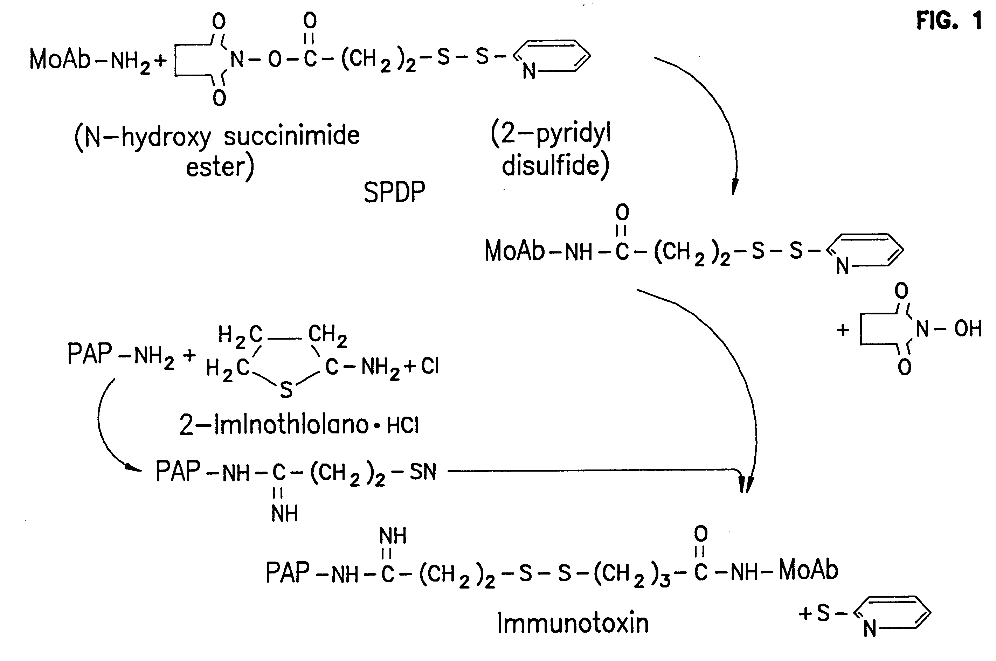

The preparation of PAP-mAb conjugates is shown in Figure 1. Monoclonal antibodies F13 (IgG$_1$, anti-CD14), G3.7 (IgG$_1$, pan-T, anti-p41 CD7), T101 (IgG$_2$a, pan-T, anti-CD5) [I. Royston et al, J. Immunol., 125, 725 (1980)] and G17-2 (IgG, anti-CD4) [J. Ledbetter et al., Mol. Immunol., 24, 1255 (1987)] were linked to pokeweed antiviral protein (PAP) by a disulfide bond using N-succinimidyl-3-(2-pyridyldithio)propionate (SPDP) as described by Uckun, Antibody, supra. PAP-mAb conjugates were purified by size exclusion chromatography on HPLC using cation exchange chromatography (Uckun, Antibody, supra. Purified PAP was reacted with a 3-fold molar excess of 2-iminothiolane for 30 min to introduce reactive sulfhydryl groups into the toxin moieties following reaction with primary amino groups as reported Uckun, Antibody, supra; See Uckun et al., Human Tumor Antigens and Specific Tumor Therapy, UCLA Symposia on Molecular and Cellular Biology, vol. 99 (Ed. Metzgar and Mitchell, Alan R. Liss, Inc., N.Y., N.Y. 1988). mAb (5 mg/ml) was first reacted with a 3-fold molar excess of SPDP (a freshly made solution of 64mM concentration in DMSO, diluted 1:10 in 40 mM sodium phosphate buffer containing 150 mM NaCl, pH7.5) for 30 min at room temperature. Modified mAb containing dithiopyridyl groups was mixed with modified PAP containing free sulfhydryl groups at a molar ratio of PAP/mAb=3/1 and incubated overnight at 4°C. Following this conjugation reaction, PAP-mAb conjugates were purified as described in Uckun et al., J. of Exp. Med.:163, 347-368 (1986); Uckun, Antibody, supra; and Uckun, Human Tumor Antigens and Specific Tumor Therapy UCLA symposia on Molecular and Cellular Bio. supra, by size exclusion and cation exchange chromatography using a computer controlled HPLC system (System Gold Beckman). PAP-mAb conjugates, F13-PAP, G3.7-PAP, T101-PAP and G17-2-PAP were separated from unconjugated PAP by size exclusion chromatography on HPLC and from unconjugated monoclonal antibody by cation exchange chromatography, as described by Uckun, Antibody Immunoconjugates and Radiopharmaceuticals, V 1:, pp 247-262 (1988).

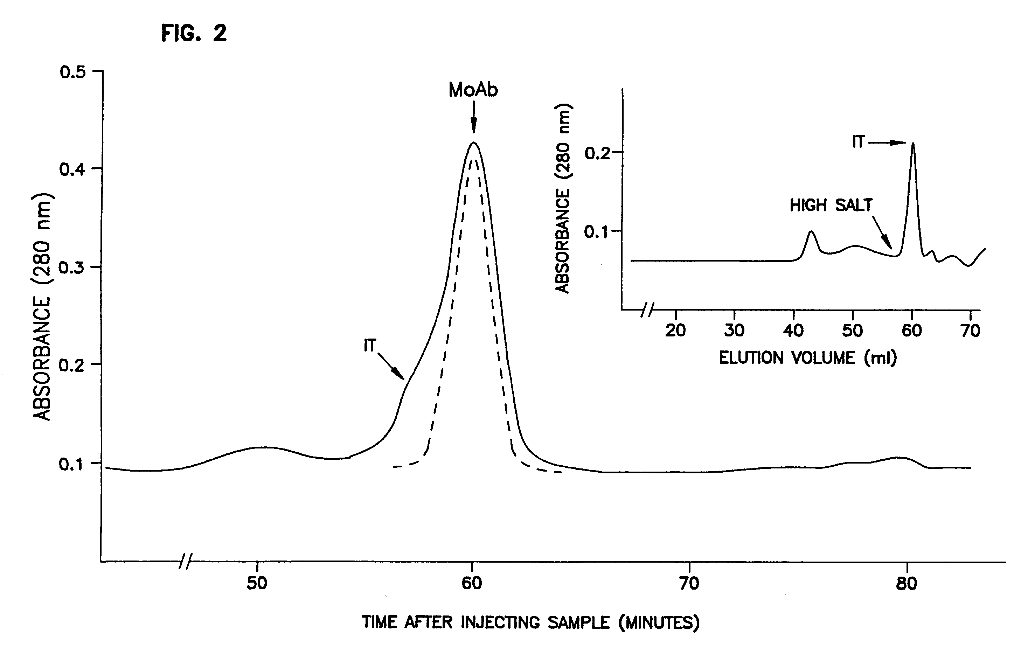

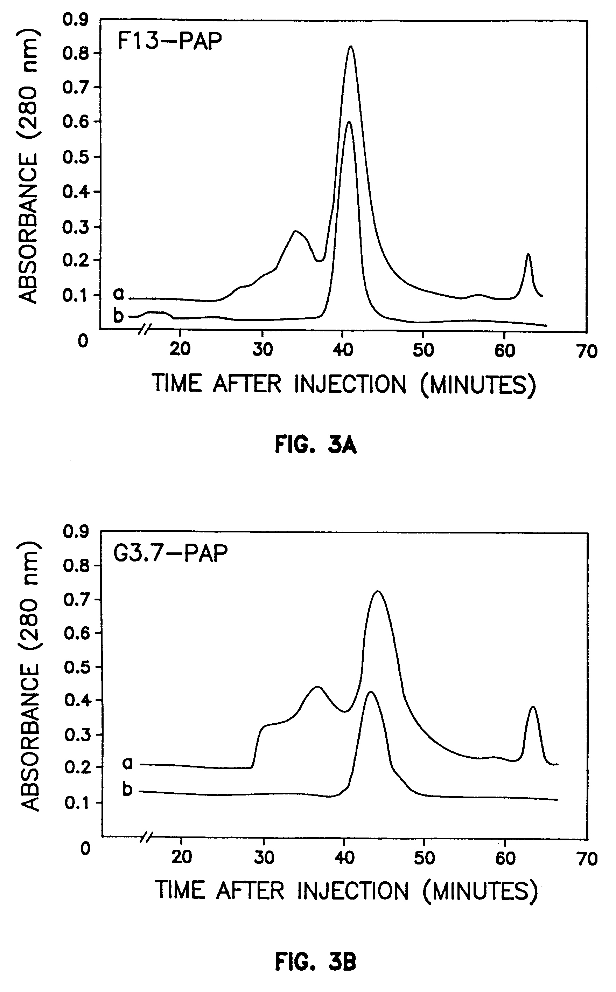

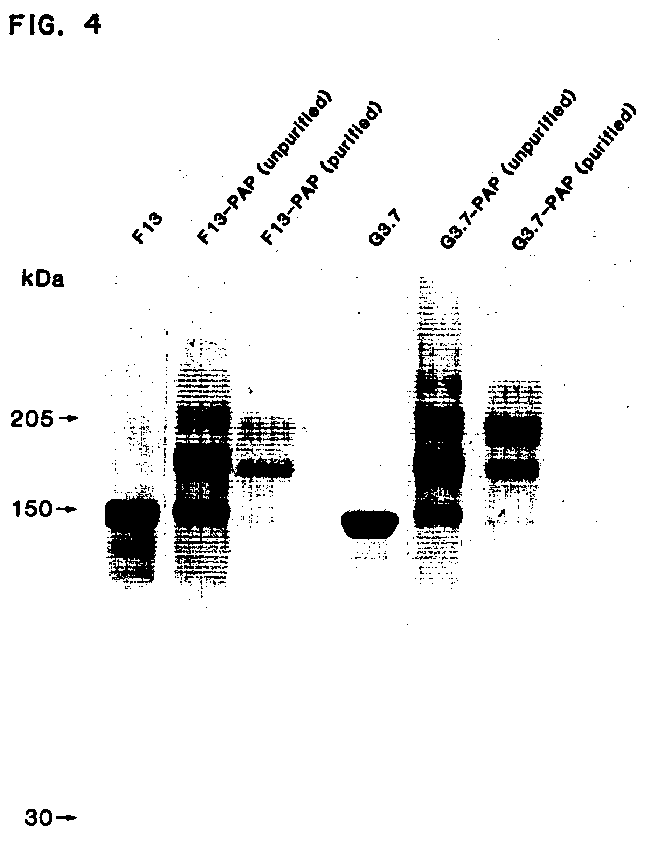

The purified PAP-mAb conjugates were evaluated in vitro with respect to purity and composition by SDS-PAGE (Fig. 4) and quantative HPLC (FIGS 2 and 3). No free PAP was detected on polyacrylamide gel electrophoresis under non-reducing conditions. Free antibody contamination was estimated to be less than 1% by gel scanning. The PAP-mAb conjugate preparations generated protein bands of 180kDa and 210 kDa, corresponding to conjugate species containing 1 or 2 molecules of PAP linked to 1 molecule of monoclonal antibody. An average molecular weight of 210 Kda for the PAP-mAb conjugate was used to calculate molar concentration because the PAP conjugates consisted primarily of two molecules of PAP linked to one molecule of mAb.

### III. Target Cells

Purified PAP-mAb conjugates were evaluated in vitro with respect to ribosome inhibiting activity by cell-free translation assays, cellular protein synthesis, clonogenic proliferation assays, $^3$H-thymidine incorporation into cells and inhibiting affect on HIV production using target cells expressing the respective antigens. As targets, HIV infected U937 monocyte and CD4$^+$ T-cells from blood, were used. Controls included (a) uninfected target cells (U937, CD4$^+$ T-cells, Nalm-6, bone marrow cells, etc.) as well as (2) target cells treated with control antibody conjugates or unconjugated PAP. Human monocyte cell line U937 (ATCC No. CRL 1593) was used.

### IV. EVALUATION OF CYTOTOXICITY OF PAP-MONOCLONAL ANTIBODY CONJUGATES

PAP-mAb conjugates were evaluated for their ability to inactivate protein synthesis in a cell-free translation system in which rat liver ribosomes and a polyuridylic acid were employed by the method of D.B. Cawley et al., Biochemistry, 17, 2648 (1979) (Table 1). Results in Table 1 show that PAP inhibits cell free translation and that PAP-monoclonal antibody conjugates containing mAb to CD19, CD14 or CD7 retain the ability to inhibit cell free translation. The cell-type specific cytotoxicity of these PAP-mAb conjugates against CEM, U937 and bone marrow stem cells was analyzed using clonogenic assays or serial dilution clonogenic assays as described in Uckun, Cancer Res. 45: 69-75, (1985); Uckun, J. of Immunology, 135: 3516-3522, (1985).

We have used two different complementary assay systems to measure cell replication in order to determine the cytotoxic effects of PAP-mAb conjugates. First, tritiated thymidine ($^3$H-TdR) incorporation assays which measure DNA synthesis and short-term replication of cells were used to determine the cytotoxicity of the target cells. This assay system is very sensitive to detect cell inhibitory effects, but it does not differentiate between transient inhibition vs. permanent inhibition (i.e., cell kill). Therefore, long-term clonogenic assays were used to determine the actual cell death after treatment with PAP-mAb conjugates.

Both assay systems yielded complementary data demonstrating a significant "therapeutic index" for inhibition of HIV replication by PAP-mAb conjugates [therapeutic index is defined as the ratio of the concentration of PAP-mAb conjugate which inhibits 90% (or 50%) HIV replication vs. the concentration of PAP-mAb conjugate which inhibits 90% (or 50%) of cell replication (see Table 3)].

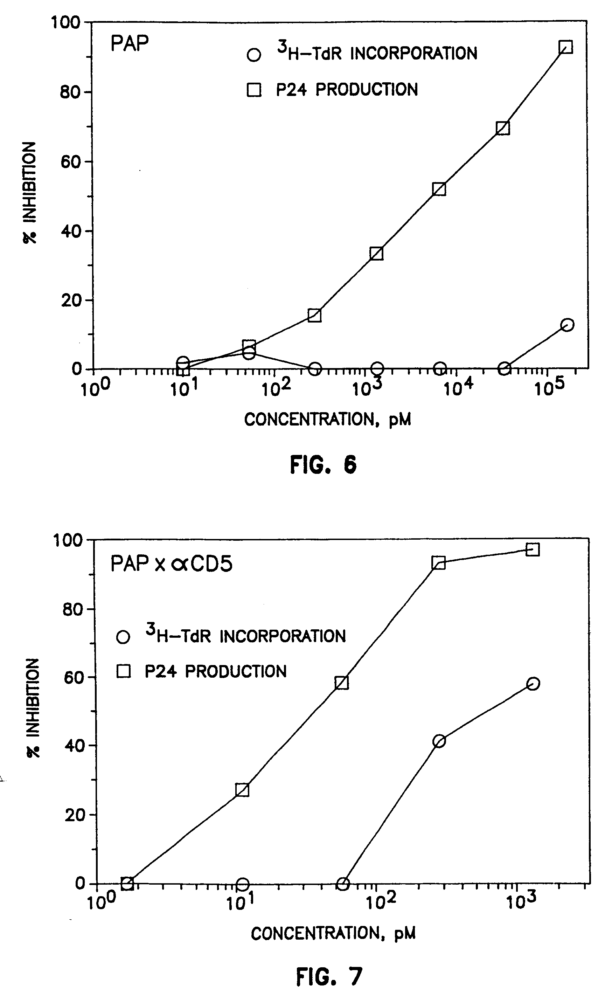

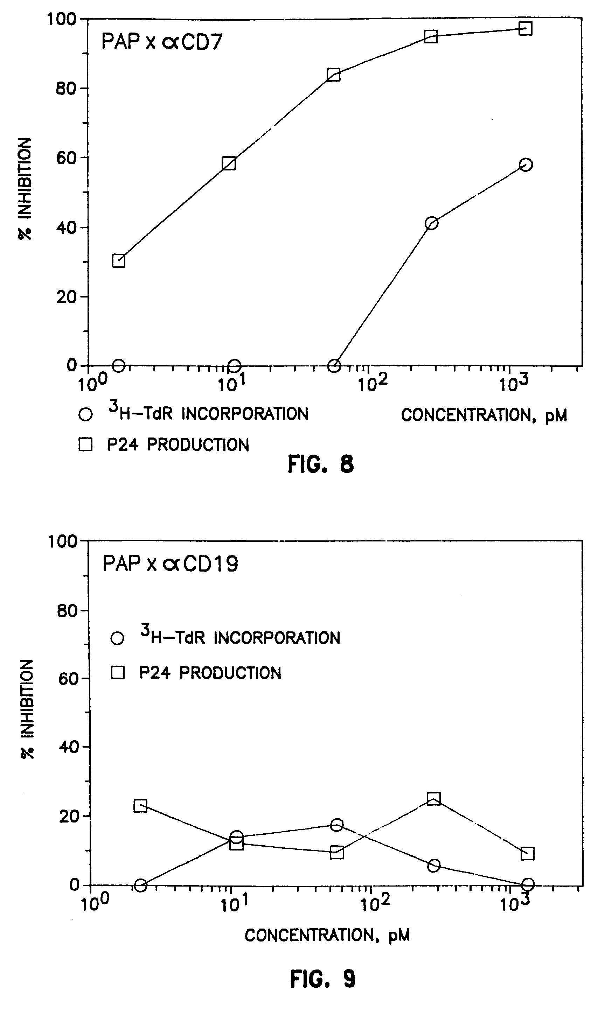

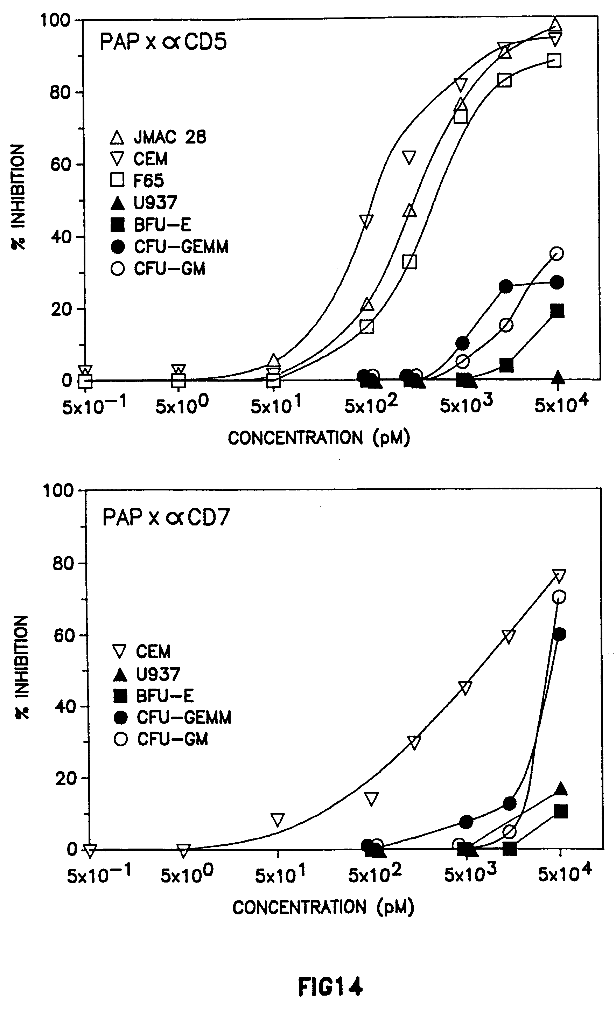

Table 3 and Figures 7, 8 and 10 show that 650 pM of PAP-anti-CD5, 600 pM of PAP-anti-CD7 and 2000 pM of PAP-anti-CD4 were required to inhibit replication of CD4$^+$ T-cells by 50%. Figure 9 shows that concentrations up to 1100 pM PAP-anti-CD19, which does not react with CD4$^+$ T-cells, did not cause more than 20% inhibition of replication of CD4$^+$ T-cells. As will be shown hereinafter (section V), when compared to the amount of PAP-monoclonal antibody conjugate required to inhibit virus replication by 50%, levels of PAP-monoclonal antibody conjugate at least 25 times less and preferably at least 100 times less than that required to inhibit cell replication by 50% were observed. Further, comparing clonogenic cell inhibition studies as seen in Fig. 14 with virus inhibition by PAP-monoclonal antibody conjugates as seen in Table 3 and Figs. 7, 8, and 10, PAP-monoclonal antibody conjugates exhibited inhibition of substantially all virus replication at levels of at least 100 fold less than levels of conjugate required for 90% inhibition of cell replication.

Fig. 14 shows the selectivity of PAP-mAb conjugates. For example: PAP-anti-CD5 inhibits clonogenic growth of CD5+ CEM (a CD4+ T-cell line), gMAC 28 (a normal CD4+ T-cell clone), and F65 (a normal CD4+ T-cell clone) cells but it does not inhibit CD5− cells such as U937 monocytes or normal bone marrow progenitor cells BFU-E, CPU-GM, or CFU-GEMM. Thus, these results show that PAP-mAb conjugates are specifically cytotoxic for target cells expressing antigen for which mAb is directed. However, PAP-mAb concentrations required to selectively inhibit target cell replication are much higher than concentrations needed to inhibit HIV replication (Table 2, 3, Figs. 7, 8).

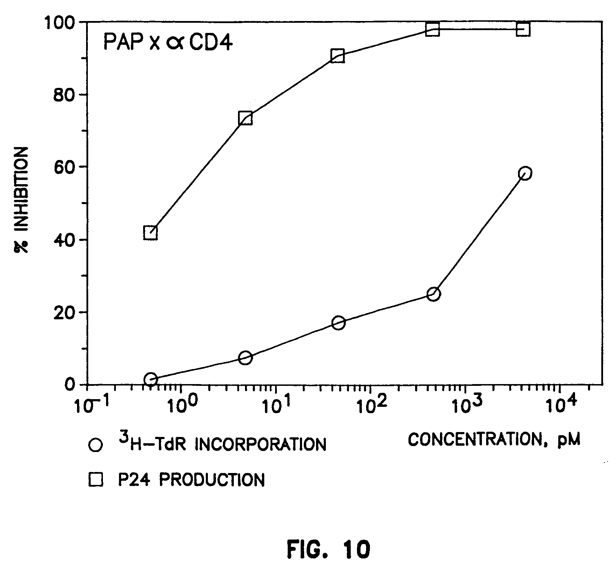

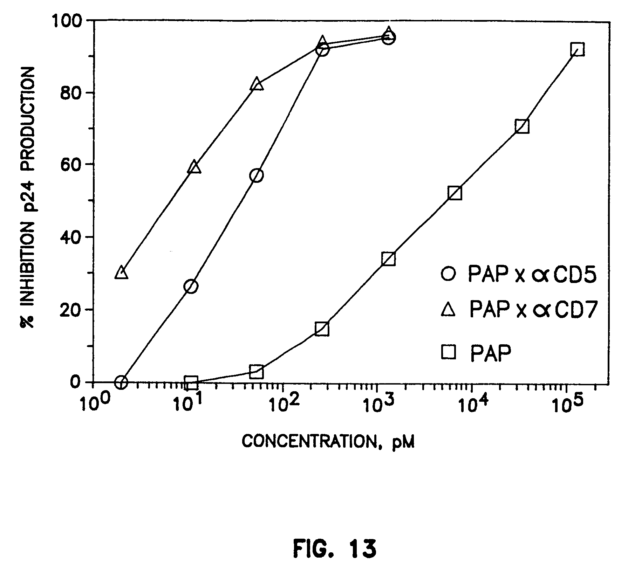

The PAP-mAb conjugates at doses required for inhibition of HIV replication in target cells are expected to elicit minimal or no toxicity to non-target cells (as defined by the lack of the relevant target antigen recognized by the mAb moiety of the PAP-mAb conjugate), including normal bone marrow progenitor cell populations since (1) less than 40% inhibition of U937, CFU-GM, BFU-E, or CFU-GEMM cells which are CD5 antigen negative was observed at highest concentrations of PAP-anti-CD5 conjugate and (2) less than 20% inhibition of U937 or BFU-E cells which are CD7 antigen negative was observed at the highest concentrations of PAP-$\alpha$CD7 conjugate (see Fig. 14). A level of inhibition which is less than 50% is not significantly different from "no inhibition" in this assay system [Uckun et al., Cancer Research 45, 69-75 (1985)]. Notably, the highest concentration of PAP-anti-CD5 or PAP-anti-CD7 tested, i.e., $5 \times 10^4$ pM, is 50-500 fold higher than the concentrations of PAP-$\alpha$CD5 or PAP-$\alpha$CD7 which are required for >90% inhibition of HIV replication, as determined by inhibition of p24 production in CD4+ T-cells (see Figs. 7, 8, 13). Thus, Fig. 14 indicates that PAP-mAb conjugates are cytotoxic to target cells expressing the relevant surface antigens when used at higher concentrations. For example, PAP-$\alpha$CD5 inhibited the proliferation of CD5 antigen positive CEM, of GMAC28, and F65 cells by $\geqq$90% at $5 \times 10^4$ pM and PAP-$\alpha$CD7 inhibited the proliferation of CD7 antigen positive CEM, CFU-GEMM, and CFU-GM cells by 50-70% at $5 \times 10^4$ pM. This concentration is 50-500 fold higher than the concentration of either conjugate required to inhibit >90% of HIV replication in CD4+ T-cells (see Figs. 7, 8, 13). PAP-anti-CD4 at 15,000 pM caused only 2%, 5.5% and 6% inhibition of colony formation by bone marrow progenitor cells BFU-E, CFU-GM, and GEMM respectively (data not shown). This concentration is about 3,000 times higher than the concentration of PAP-anti-CD4 conjugate required to inhibit 90% of HIV-1 replication in CD4+ T-cells (Fig. 10).

Fig. 7 demonstrates that PAP-$\alpha$CD5 conjugate inhibits >90% of HIV replication in CD4+ T-cells at $3 \times 10^2$ pM. At this concentration, PAP-$\alpha$CD5 inhibited the short-term replication (as measured by $^3$H-thymidine incorporation) of CD4+ T-cells (Fig. 7) or the long-term replication (as measured by clonogenic assays) of CEM, GMAC28, and F65 cells, which all express the CD5 surface antigen, by 40% or less.

Similarly, Fig. 8 demonstrates that PAP-$\alpha$CD7 conjugate inhibits >90% of HIV replication in CD4+ T-cells at $3 \times 10^2$ pM. At this concentration, it inhibited 40% of short-term replication of CD4+ T-cells, and <20% of long-term replication of CEM, CFU-GEMM, or CFU-GM cells which all express the CD7 surface antigen (Fig. 14). Similarly, Fig. 10 demonstrates that PAP-anti-CD4 inhibits >90% of HIV-1 replication in CD4+ T-cells at 30 pM. At this concentration, it inhibited 20% of short-term replication of CD4+ T-cells. At 30 pM, PAP-anti-CD4 there was no inhibition of CFU-GM, BFU-E or GEMM. These findings illustrate that PAP-mAb conjugates can inhibit HIV replication in CD4+ T-cells without inhibiting replication of uninfected CD4+ T-cells or colony formation by bone marrow cells.

TABLE 1

| Inhibitory Effects of PAP Monoclonal Antibody Conjugates on Cell-Free Translation | |
|---|---|
| | $IC_{50}$ (pM) |
| B43-PAP (anti-CD19) | 21 |
| F13-PAP (anti-CD14) | 14 |
| G3.7-PAP (anti-CD7) | 39 |
| G17-2-PAP (anti-CD4) | 25 |
| PAP | 8 |
| The effect of PAP antibody conjugates on cell-free translation was analyzed under reducing conditions in a cell-free translation system which consists of a nuclease-treated rabbit reticulocyte lysate with the ability to translate messenger RNA into protein. Protein synthesis was measured as $^3$H-leucine incorporation into alkali-resistant TCA precipitable material. $IC_{50}$ = concentration which inhibits 50% of protein synthesis. | |

### V. Evaluation of PAP-Monoclonal Antibody Conjugate Effects on HIV Production in Monocytes and T-Cells Infected in vitro with HIV-1 and on HIV Production in T-cells From Infected Patients.

T-cells and U937 monocytes exposed to HIV-1 following treatment with PAP-monoclonal antibody conjugates were monitored for the production of HIV p24 (gag) antigen in culture supernatants using an HIV-1 antigen capture enzyme-linked immunosorbent assay (ELISA). (Genetic Systems Corp.) PAP-mAb conjugates were analyzed for their anti-HIV activity over a 5 log dose range. Sublethal doses which will effectively abrogate viral replication in CD4$^+$ T-cells without killing uninfected CD4$^+$ T-cells were studied.

U937 cells, cultured in RPMI containing 10% FCS, were pelleted and then resuspended in fresh RPMI containing 10% FCS at a density of $1 \times 10^5$ cells/ml. PAP or PAP-mAb conjugate was freshly prepared at 4 times the desired final concentration in the same medium. 0.1 ml of the cell suspension and 0.05 ml of drug or medium were added to each of 8 replicate wells of 96-well flat-bottom plates. On the following day, 0.05 ml of serially diluted LAV-1 isolate of HIV-1 was added to each well. One day after infection, the cells were washed twice to remove unadsorbed virus. New media containing the designated drug concentrations of PAP or PAP-mAb conjugate was added to each well. Seven days after infection 0.1 ml of supernatant was removed from each well and assayed for HIV p24 (gag) antigen by antigen-capture ELISA (Genetic Systems Inc.). Cells treated at various concentrations are reported in Table 2. Table 2 shows that both PAP and PAP-anti-CD14 inhibit HIV-p24 production in the HIV-infected U937 monocyte cells and that the PAP-anti-CD14 conjugate is more effective completely abrogating HIV replicated at concentrations as low as 150 pM.

For the study of effects of PAP and PAP-mAb conjugates on HIV-1 replication in CD4$^+$ T-cells obtained from normal humans, $25 \times 10^6$ peripheral blood lymphocytes (PBL), isolated from fresh human blood by Ficoll density gradient centrifugation, were resuspended in 25 ml RPMI 1640 medium containing 10% normal human serum (NHS) and were incubated for one hour at 37°C in a 150 cm$^2$ tissue culture flask to remove adherent monocytes.

The nonadherent PBL were then incubated for 30' on ice in medium containing 10% NHS and a combination of the following monoclonal antibodies, each at 10 μg/ml: G10.1 (αCD8), FC-1 (αCD16) and IF5 ($\propto$CD20). To the antibody treated cells was added Pel Freeze 3-4 week rabbit complement at a final dilution of 1:4. The cells were incubated with complement for 1 hr at 37°C and then dead cells were eliminated on Ficoll. The viable cells were incubated overnight in RPMI medium containing 10% NHS and 3 μg/ml phytohemagglutinin (PHA).

PHA was then removed by washing the cells with medium and $2 \times 10^6$ cells were resuspended with 0.5 ml medium containing various concentrations of PAP or PAP-mAb conjugates or in medium alone and then incubated for 4 hours at 37°C. Then $5 \times 10^4$ Tissue Culture Infectious Doses$_{50}$ (TCID$_{50}$) of the LAV-1 isolate of HIV-1 were added to each tube.

After 2 hours non-cell-bound virus was removed by washing cells in PBS four times and the cells were resuspended in 2.0 ml medium ($1 \times 10^6$ cells/ml). Then $1 \times 10^5$ cells in 0.1 ml was added to each of 4 replicate wells of round bottom 96-well plates containing 0.1 ml of various concentrations of PAP, PAP-mAb conjugates or medium alone and 4 units/ml IL-2 (Lymphocult-T).

On day 7 supernatant was removed from walls of a duplicate plate and assayed for HIV p24 (gag) in a quantitative p24 antigen-capture ELISA (GSC). The percent inhibition of p24 production was calculated as follows:

$$1 - \frac{\text{ng/ml p24 produced by treated cells}}{\text{ng/ml p24 produced by untreated cells}} \times 100$$

Supernatants from untreated CD4$^+$ T-cells contained 1255 ng/ml of p24. The results of anti-HIV activity of PAP conjugates with mAb to CD5, CD7, or CD4, and with PAP conjugate to negative control mAb to CD19 are shown in Table 3 and Figures 7-10, 13.

Pretreatment of CD4$^+$ T-cells with unconjugated PAP at concentrations of approximately 5000 pM inhibited HIV-1 production by approximately 50% in these cells that were infected with LAV-1 isolate of HIV-1, providing unique evidence that the antiviral spectrum of PAP includes HIV-1. (Table 3, Fig. 6) PAP-anti-CD5 (T101), PAP-anti-CD7 (G3.7) reactive with T-cells, and PAP-anti-CD4 (G17-2) specifically reactive with CD4$^+$ T-cells were highly effective in inhibiting virus replication in CD4$^+$ T-cells (Figs. 7, 8, and 10, and Table 3). Control PAP conjugate containing B43/CD19 which reacts with control B-cells but not T-cells did not show 50% inhibition of HIV replication in CD4$^+$ T-cells even at 1,375 pM (Fig. 9, Table 3) which is about 220-fold higher than the concentration of PAP-anti-CD7 and about 1000 fold higher than the concentration of PAP-anti-CD4 required to inhibit HIV p24 production in CD4$^+$ T-cells by 50% (Figures 8, 10 and Table 3). Notably, the concentrations of PAP conjugated with anti-CD5, anti-CD7 or anti-CD4 inhibitory to HIV replication are much lower than those required for inhibition of cell replication (Fig. 7, 8, 10, 13 and Table 3).

For the study of the effect of PAP-anti-CD4 conjugate on HIV-1 p24 production in peripheral blood lymphocytes (PBL) from HIV-1 infected humans, PBL were isolated from fresh blood by ficoll density gradient centrifugation and resuspended at $1 \times 10^6$ cells/ml in RPMI 1640 medium containing 10% normal human serum (NHS). Then $1 \times 10^6$ cells in 1.0 ml was added to each of two replicate wells of 24-well plates containing 1 ml of various concentrations of PAP-anti-CD4 conjugate together with 1 μg/ml monoclonal antibody to CD3 (IgG1, G19.4 ATCC No. HB9536 deposited September 15, 1987) to induce replication of the PBL and activation of HIV-1 production and 4 units/ml IL-2 (Electro-Nucleonics).

On day 5, 0.1 ml aliquots of cell suspensions from each treatment were transferred to round-bottom, 96-well plates and labeled with 1.0 µCi in 0.1 ml per well of tritiated thymidine (New England Nuclear) and harvested using a cell harvester 6 hours later.

On day 7, supernatant was removed from wells and assayed for HIV-1 p24 levels in a quantitative p24 antigen-capture ELISA (Genetic Systems). The percent inhibition of p24 production was calculated as follows:

$$1 - \frac{\text{ng/ml p24 produced by treated cells}}{\text{ng/ml p24 produced by untreated cells}} \times 100$$

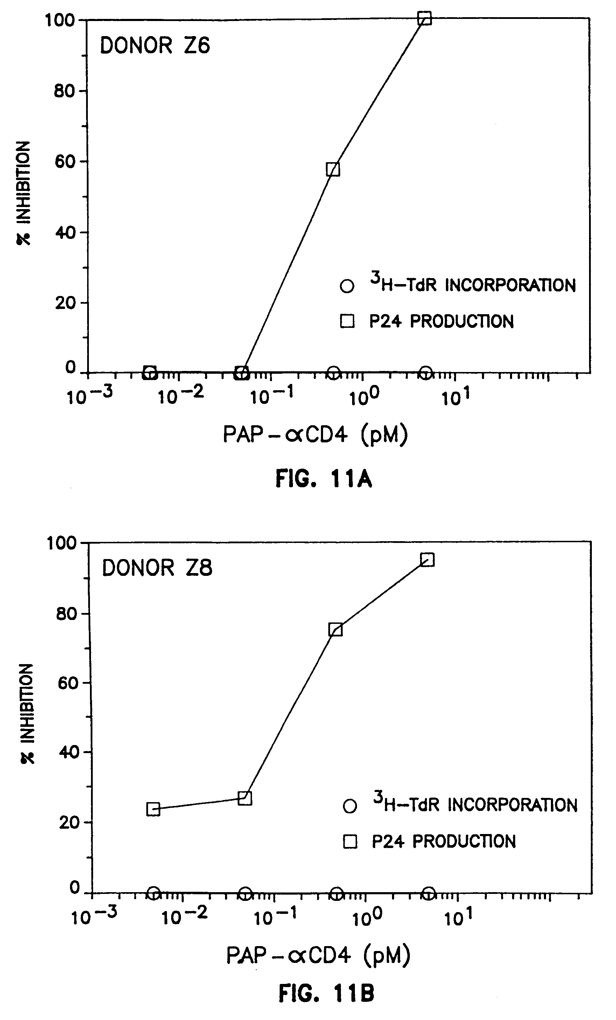

The results of the effects of PAP conjugated with mAb to CD4 on inhibiting HIV-1 production in PBL from two infected patients are shown in Fig. 11

Figure 11 shows that treatment of PBL from HIV-1 infected patients with mAb G19.4 to CD3 induces HIV-1 production. The p24 concentrations in supernatants from anti-CD3 activated PBL, not treated with PAP-mAb CD4, from donors Z6 and Z8, were 0.4 and 6.6 ng/ml, respectively. However, a concentration of about 0.5 pM PAP-anti-CD4 inhibited HIV-1 replication (p24) by 50%. A concentration of about 5 pM PAP-anti-CD4 inhibited p24 production by almost 100% but did not inhibit cell replication of the patients' PBL.

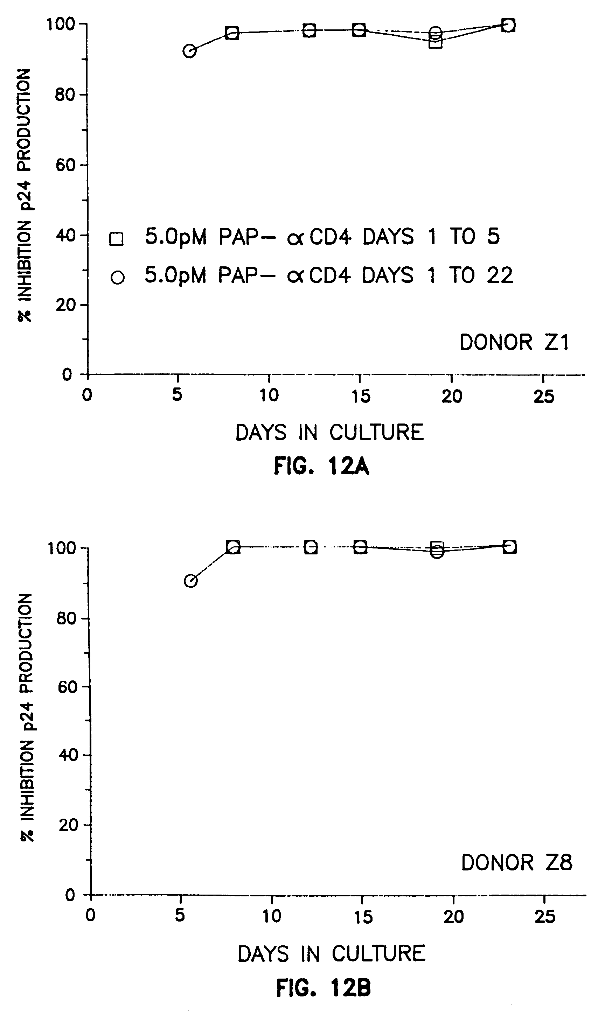

Figure 12 shows that the anti-HIV effect of PAP-anti-CD4 (5.0 pM) on two patients' anti-CD3 activated PBL lasted at least 22 days even when the cells were washed out of PAP-anti-CD4 on day 5.

These findings indicate that monoclonal antibody conjugates of pokeweed antiviral protein can be used for selective inhibition of HIV production in cells bearing the relevant target surface antigens against which the monoclonal antibody is directed without the conjugates inhibiting cell replication. Notably, PAP-anti-CD4 is not only very potent in inhibiting HIV-1 replication in normal cells infected in vitro with HIV-1, but is also very potent at inhibiting HIV-1 production in patients' PBL and the effect lasts at least several weeks (Figs. 11 and 12). PAP-mAb conjugates have an in vivo half-life of about 16-18 hours in mice and 6-10 hours in non-human primates compared to the 5-10 minute half-life of PAP and are effective at inhibiting HIV-1 production at non-toxic concentrations which are at least 200-fold less than the effective concentration of PAP.

To cause inhibition of HIV replication in U937 monocyte cells PAP-anti-CD14 was effective below 150 pM whereas $3 \times 10^5$ pM non-conjugated PAP was required (Table 2). To cause 50% inhibition of HIV replication in CD4$^+$ T-cells with PAP conjugated to anti-CD5, concentration of 32 pM was required (Figure 7); with PAP anti-CD7 (Figure 8) a concentration of 6 pM was required, and with PAP-anti-CD4 a concentration of 1 pM was required (Fig. 10 and Table 3). In contrast, a concentration of 5000 pM of non-conjugated PAP was required to inhibit HIV replication by 50% (see Fig. 6 and Table 3). Thus, with PAP-mAb conjugates 25-5000 times less is required than PAP alone to inhibit HIV replication (Figs. 8, 10, and Table 3). By comparison, the control PAP-mAb conjugate PAP-anti-CD19 which does not react with CD4$^+$ T-cells since it is specific for B-cells, neither inhibited the proliferation of CD4$^+$ T-cells, nor did it inhibit HIV replication at concentrations as high as 1,375 pM (Table 3, Fig. 9). These results show that PAP can be selectively targeted to inhibit HIV replication in CD4$^+$ cells by conjugating PAP to mAb reactive with CD4$^+$ cells. Results shown in Figs. 11 and 12 show that PAP-anti-CD4 conjugate is also very potent and long-lasting in inhibiting HIV-1 production in PBL from patients infected with HIV-1.

TABLE 2

| INHIBITION OF HIV-1 PRODUCTION IN HUMAN U-937 MONOCYTE CELLS TREATED WITH PAP-ANTI-CD14 OR WITH NON-CONJUGATED PAP | |
| --- | --- |
| U937 Cells Treated with: PAP-αCD14 Conjugate Concentration (pM) | Number of HIV p24+ Wells Incorporation into |
| None | 14/16 |
| $2.8 \times 10^1$ | 4/16 |
| $1.4 \times 10^2$ | 0/8 |
| $6.9 \times 10^2$ | 0/8 |
| PAP Alone Concentration (pM) | |
| None | 4/8 |
| $3.3 \times 10^2$ | 4/8 |
| $3.3 \times 10^3$ | 3/8 |
| $3.3 \times 10^4$ | 3/8 |
| $3.3 \times 10^5$ | 1/8 |

TABLE 3

| Improved Anti-HIV Activity of PAP After Conjugation with mAb to CD5, CD7 or CD4 | | |
|---|---|---|
| CD4+T Cells Treated With | Anti-HIV Inhibitory Dose$_{50}$ Concentration (pM) | Anti-Proliferative Inhibitory Dose$_{50}$ Concentration (pM) |
| PAP | 5,000 | >200,000 |
| PAP$_\alpha$CD5 | 32 | 650 |
| PAP$_\alpha$CD7 | 6 | 600 |
| PAP$\alpha$CD4 | 1 | 2,000 |
| PAP$_\alpha$CD19 | >1,375 | >1,375 |
| Inhibitory dose$_{50}$ (ID$_{50}$) = concentration required to inhibit cell proliferation ($^3$H-TdR incorporation) of CD4+ T-cells by 50% and concentration required to inhibit HIV replication (p24 antigen production) in CD4+ T-cells by 50%. Because the highest PAP $\alpha$CD19 conjugate dose tested was 1,375 pM, the ID$_{50}$ of this control conjugate could not be determined. The anti-proliferation ID$_{50}$ of PAP also exceeds the highest dose tested ($2 \times 10^5$ pM). PAP$\alpha$CD19 does not react with CD4+ T-cells and was chosen as the negative control. | | |

**Claims**

**Claims for the following Contracting States : AT, BE, CH, DE, DK, FR, GB, IT, LI, LU, NL, SE**

1. An antiviral composition, comprising pokeweed antiviral protein conjugate effective to inhibit virus replication in virus-infected mammalian cells without detectable cytotoxicity and a human monoclonal antibody reactive with a non-virus associated antigen present at the surface of such mammalian cells,
**characterized in that**
said monoclonal antibody is a CD4 or CD5 antigen monoclonal antibody.

2. The composition of claim 1, characterized in that said antibody is monoclonal antibody T101/CD5, 10.2/CD5 or G17-2/CD4.

3. The composition of claim 2, characterized in that said monoclonal antibody T101/CD5, 10.2/CD5 or G17-2/CD4 and pokeweed antiviral protein conjugate is effective to inhibit HIV or HTLV replication in HIV- oder HTLV-infected mammalian cells.

4. A method for inhibiting replication of HIV in mammalian cells,
**characterized by**
treating a cell culture including HIV-infected human T-cells with an amount of a human CD4 antigen antibody and pokeweed antiviral protein conjugate composition or human CD5 antigen antibody and pokeweed antiviral protein conjugate composition or a mixture thereof effective to inhibit replication of said HIV without detectable cytotoxicity, said antibody of said conjugate being reactive with a non-virus-associated antigen present at the surface of said HIV-infected cells.

5. The method of claim 4, characterized in that said antibody is monoclonal antibody G17-2/CD4 or another antibody where binding is substantially blocked by G17-2 and which blocks the binding of G17-2/CD4.

6. The method of claim 5, characterized in that said antibody is monoclonal antibody 10.2/CD5 or another antibody where binding is substantially blocked by 10.2 and which blocks the binding of 10.2/CD5.

7. A method for inhibiting replication of HIV in mammalian cells,
**characterized by**
stimulating peripheral blood lymphocytes obtained from HIV-1 infected patients with an amount of an agent effective to induce cell proliferation and HIV replication; and
treating said peripheral blood lymphocytes with an amount of an antibody and pokeweed antiviral protein conjugate composition effective to inhibit replication of said HIV, wherein said antibody is a CD4 or CD5 antigen monoclonal antibody reactive with a non-virus associated antigen present at the surface of such mammalian cells.

8. The method of claim 7, characterized in that said cell proliferation and HIV replication agent is a CD3 antibody.

9. The method of claim 7, characterized in that said cell proliferation and HIV replication agent is phytohemagglutin.

10. The method of claim 8, characterized in that said CD3 antibody is G19-4/CD3.

**Claims for the following Contracting State : ES**

1. A method for making an antiviral composition comprising pokeweed antiviral protein conjugate effective to inhibit virus replication in virus-infected mammalian cells without detectable cytotoxicity and a human monoclonal antibody reactive with a non-virus associated antigen present at the surface of such mammalian cells, the method comprising conjugating said pokeweed antiviral protein with said human monoclonal antibody,
**characterized in that**
said monoclonal antibody is a CD4 or CD5 antigen monoclonal antibody.

2. The method of claim 1, characterized in that said antibody is monoclonal antibody T101/CD5, 10.2/CD5 or G17-2/CD4.

3. The method of claim 2, characterized in that said monoclonal antibody T101/CD5, 10.2/CD5 or G17-2/CD4 and pokeweed antiviral protein conjugate is effective to inhibit HIV or HTLV replication in HIV- oder HTLV-infected mammalian cells.

4. A method for inhibiting replication of HIV in mammalian cells,
**characterized by**
treating a cell culture including HIV-infected human T-cells with an amount of a human CD4 antigen antibody and pokeweed antiviral protein conjugate composition or human CD5 antigen antibody and pokeweed antiviral protein conjugate composition or a mixture thereof effective to inhibit replication of said HIV without detectable cytotoxicity, said antibody of said conjugate being reactive with a non-virus-associated antigen present at the surface of said HIV-infected cells.

5. The method of claim 4, characterized in that said antibody is monoclonal antibody G17-2/CD4 or another antibody where binding is substantially blocked by G17-2 and which blocks the binding of G17-2/CD4.

6. The method of claim 5, characterized in that said antibody is monoclonal antibody 10.2/CD5 or another antibody where binding is substantially blocked by 10.2 and which blocks the binding of 10.2/CD5.

7. A method for inhibiting replication of HIV in mammalian cells,
**characterized by**
stimulating peripheral blood lymphocytes obtained from HIV-1 infected patients with an amount of an agent effective to induce cell proliferation and HIV replication; and
treating said peripheral blood lymphocytes with an amount of an antibody and pokeweed antiviral protein conjugate composition effective to inhibit replication of said HIV, wherein said antibody is a CD4 or CD5 antigen monoclonal antibody reactive with a non-virus associated antigen present at the surface of such mammalian cells.

8. The method of claim 7, characterized in that said cell proliferation and HIV replication agent is a CD3 antibody.

9. The method of claim 7, characterized in that said cell proliferation and HIV replication agent is phytohemagglutin.

10. The method of claim 8, characterized in that said CD3 antibody is G19-4/CD3.

**Patentansprüche**

**Patentansprüche für folgende Vertragsstaaten : AT, BE, CH, DE, DK, FR, GB, IT, LI, LU, NL, SE**

1. Antivirale Zusammensetzung, mit einem Konjugat von antiviralem pokeweed(Phytolacca Americana)-Protein (PAP), wirksam, die Virusreplikation in virusinfizierten Säugetierzellen ohne meßbare Zytotoxizität zu unterdrücken und einem menschlichen monoklonalen Antikörper, reaktionsfähig mit einem nicht-viralen assoziierten, an der Oberfläche derartiger Säugetierzellen vorhandenen Antigen,
dadurch gekennzeichnet, daß der monoklonale Antikörper ein monoklonaler CD4 oder CD5 Antigen-Antikörper ist.

2. Zusammensetzung nach Anspruch 1,
dadurch gekennzeichnet, daß der Antikörper ein monoklonaler T101/CD5, 10.2/CD5 oder G17-2/CD4 Antikörper ist.

3. Zusammensetzung nach Anspruch 2,
dadurch gekennzeichnet, daß das Konjugat von monoklonalen T101/CD5, 10.2/CD5 oder G17-2/CD4 Antikörpern und PAP wirksam ist, die HIV- oder HTLV-Replikation in HIV- oder HTLV-infizierten Säugetierzellen zu unterdrücken.

4. Verfahren zur Unterdrückung der Replikation von HIV in Säugetierzellen,
dadurch gekennzeichnet, daß eine Zellkultur einschließlich HIV-infizierten menschlichen T-Zellen mit einer Menge einer Zusammensetzung eines Konjugats menschlicher CD4-Antigen-Antikörper und PAP oder einer Zusammensetzung eines Konjugats menschlicher CD5-Antigen-Antikörper und PAP oder einer Mischung davon behandelt wird, wirksam, die Replikation des HIV ohne meßbare Zytotoxizität zu unterdrücken, wobei der Antikörper des Konjugats mit einem nicht-viralen, assoziierten, an der Oberfläche der HIV-infizierten Zellen vorhandenen Antigen reaktionsfähig ist.

5. Verfahren nach Anspruch 4,
dadurch gekennzeichnet, daß der Antikörper der monoklonale Antikörper G17-2/CD4 oder ein anderer Antikörper ist, bei dem Bindungen im wesentlichen durch G17-2 blockiert werden und welcher die Bindung von G17-2/CD4 blockiert.

6. Verfahren nach Anspruch 5,
dadurch gekennzeichnet, daß der Antikörper der monoklonale Antikörper 10.2/CD5 oder ein anderer Antikörper ist, bei welchem die Bindung im wesentlichen durch 10.2 blockiert wird und welcher die Bindung von 10.2/CD5 blockiert.

7. Verfahren zum Unterdrücken der Replikation von HIV in Säugetierzellen,
dadurch gekennzeichnet, daß periphere, von HIV-1 infizierten Patienten erhaltene Blut-Lymphozyten mit einer Menge eines Wirkstoffes stimuliert werden, wirksam, die Zell-Proliferation und die HIV-Replikation zu induzieren; und
daß die peripheren Blutlymphozyten mit einer Menge einer Zusammensetzung eines Konjugats von Antikörpern und PAP behandelt werden, wirksam, die Replikation des HIV zu unterdrücken, in der der Antikörper ein monokionaler CD4 oder CD5-Antigen-Antikörper ist, welcher mit einem nicht-viralen, assoziierten, an der Oberfläche derartiger Säugetierzellen vorhandenen Antigen reaktionsfähig ist.

8. Verfahren nach Anspruch 7,
dadurch gekennzeichnet, daß der Zellproliferations- und HIV-Replikations-Wirkstoff ein CD3-Antikörper ist.

9. Verfahren nach Anspruch 7,
dadurch gekennzeichnet, daß der Zellproliferations- und HIV-Replikations-Wirkstoff Phytohämagglutinin ist.

10. Verfahren nach Anspruch 8,
dadurch gekennzeichnet, daß der CD3-Antikörper G19-4/CD3 ist.

**Patentansprüche für folgenden Vertragsstaat : ES**

1. Verfahren zur Herstellung einer antiviralen Mischung, mit einem Konjugat von antiviralem pokeweed(Phytolacca Americana)-Protein (PAP), wirksam, die Virusreplikation in virusinfizierten Säugetierzellen ohne meßbare Zytotoxizität zu unterdrücken und einem menschlichen monoklonalen Antikörper, welcher mit einem nicht-viralen assoziierten, an der Oberfläche derartiger Säugetierzellen vorhandenen Antigen reaktionsfähig ist, wobei das Verfahren die Konjugation des PAP mit dem menschlichen monokionalen Antikörper umfaßt,
dadurch gekennzeichnet, daß der monoklonale Antikörper ein monoklonaler CD4- oder CD5-Antigen-Antikörper ist.

2. Verfahren nach Anspruch 1,
dadurch gekennzeichnet, daß der Antikörper ein monokionaler T101/CD5, 10.2/CD5 oder G17-2/CD4-Antikörper ist.

3. Verfahren nach Anspruch 2,
dadurch gekennzeichnet, daß das Konjugat von monokionalen T101/CD5, 10.2/CD5 oder G17-2/CD4-Antikörpern und PAP wirksam ist, die HIV- oder HTLV-Replikation in HIV- oder HTLV-infizierten Säugetierzellen zu unterdrücken.

**4.** Verfahren zur Unterdrückung der Replikation von HIV in Säugetierzellen,
dadurch gekennzeichnet, daß eine Zellkultur einschließlich HIV-infizierten menschlichen T-Zellen mit einer Menge einer Zusammensetzung eines Konjugats menschlicher CD4-Antigen-Antikörper und PAP oder einer Zusammensetzung eines Konjugats menschlicher CD5-Antigen-Antikörper oder einer Mischung davon behandelt wird, wirksam, PAP die Replikation des HIV ohne meßbare Zytotoxizität zu unterdrücken, wobei der Antikörper des Konjugats mit einem nicht-viralen, assoziierten, an der Oberfläche der HIV-infizierten Zellen vorhandenen Antigen reaktionsfähig ist.

**5.** Verfahren nach Anspruch 4,
dadurch gekennzeichnet, daß der Antikörper der monoklonale Antikörper G17-2/CD4 oder ein anderer Antikörper ist, bei dem Bindungen im wesentlichen durch G17-2 blockiert werden und welcher die Bindung von G17-2/CD4 blockiert.

**6.** Verfahren nach Anspruch 5,
dadurch gekennzeichnet, daß der Antikörper der monoklonale Antikörper 10.2/CD5 oder ein anderer Antikörper ist, bei welchem die Bindung im wesentlichen durch 10.2 blockiert wird und welcher die Bindung von 10.2/CD5 blockiert.

**7.** Verfahren zum Unterdrücken der Replikation von HIV in Säugetierzellen,
dadurch gekennzeichnet, daß periphere, von HIV-1 infizierten Patienten erhaltene Blut-Lymphozyten mit einer Menge eines Wirkstoffes stimuliert werden, wirksam, die Zell-Proliferation und die HIV-Replikation zu induzieren; und
daß die peripheren Blutlymphozyten mit einer Menge einer Zusammensetzung eines Konjugats von Antikörpern und PAP behandelt werden, wirksam, die Replikation des HIV zu unterdrücken, in der der Antikörper ein monoklonaler CD4 oder CD5-Antigen-Antikörper ist, welcher mit einem nicht-viralen, assoziierten, an der Oberfläche derartiger Säugetierzellen vorhandenen Antigen reaktionsfähig ist.

**8.** Verfahren nach Anspruch 7,
dadurch gekennzeichnet, daß der Zellproliferations- und HIV-Replikations-Wirkstoff ein CD3-Antikörper ist.

**9.** Verfahren nach Anspruch 7,
dadurch gekennzeichnet, daß der Zellproliferations- und HIV-Replikations-Wirkstoff Phytohämagglutinin ist.

**10.** Verfahren nach Anspruch 8,
dadurch gekennzeichnet, daß der CD3-Antikörper G19-4/CD3 ist.

**Revendications**

**Revendications pour les Etats contractants suivants : AT, BE, CH, DE, DK, FR, GB, IT, LI, LU, NL, SE**

**1.** Composition antivirale, comprenant un conjugué de protéine antivirale pokeweed efficace pour inhiber la réplication d'un virus dans des cellules de mammifère infectées par un virus, sans cytotoxicité décelable, et un anticorps monoclonal réactif avec un antigène non associé au virus présent à la surface de ces cellules de mammifère, caractérisée en ce que ledit anticorps monoclonal est un anticorps monoclonal antiantigène CD4 ou CD5.

**2.** Composition selon la revendication 1, caractérisée en ce que ledit anticorps est un anticorps monoclonal T101/CD5, 10.2/CD5 ou G17-2/CD4.

**3.** Composition selon la revendication 2, caractérisée en ce que ledit anticorps monoclonal T101/CD5, 10.2/CD5 ou G17-2/CD4 et conjugué de protéine antivirale pokeweed est efficace pour inhiber la réplication de HIV ou de HTLV dans des cellules de mammifère infectées par HIV ou par HTLV.

**4.** Procédé pour inhiber la réplication de HIV dans des cellules de mammifère, caractérisé par le traitent d'une culture cellulaire comprenant des cellules T humaines infectées par HIV avec une quantité d'une composition d'anticorps antiantigène CD4 humain et de conjugué de protéine antivirale pokeweed ou d'une composition d'anticorps antiantigène CD5 humain et de conjugué de protéine antivirale pokeweed, ou un mélange de celles-ci, efficace pour inhiber la réplication dudit HIV sans cytotoxicité décelable, ledit anticorps dudit conjugué étant réactif avec un antigène non associé au virus présent à la surface desdites cellules infectées par HIV.

**5.** Procédé selon la revendication 4, caractérisé en ce que ledit anticorps est un anticorps monoclonal G17-2/CD4 ou un autre anticorps où la liaison est essentiellement bloquée par G17-2 et qui bloque la liaison de G17-2/CD4.

**6.** Procédé selon la revendication 5, caractérisé en ce que ledit anticorps est un anticorps monoclonal 10.2/CD5 ou un autre anticorps où la liaison est essentiellement bloquée par 10.2 et qui bloque la liaison de 10.2/CD5.

**7.** Procédé pour inhiber la réplication de HIV dans des cellules de mammifère, caractérisé par
la stimulation des lymphocytes du sang périphérique obtenus à partir de patients infectés par HIV-1 avec une quantité d'un agent efficace pour provoquer une prolifération cellulaire et une réplication de HIV; et
le traitement desdits lymphocytes du sang périphérique avec une quantité d'une composition d'anticorps et de conjugué de protéine antivirale pokeweed efficace pour inhiber la réplication dudit HIV, dans lequel ledit anticorps est un anticorps monoclonal antiantigène CD4 ou anti-CD5 réactif avec un antigène non associé au virus présent à la surface de ces cellules de mammifère.

**8.** Procédé selon la revendication 7, caractérisé en ce que ledit agent de prolifération cellulaire et de réplication de HIV est un anticorps CD3.

**9.** Procédé selon la revendication 7, caractérisé en ce que ledit agent de prolifération cellulaire et de réplication de HIV est la phytohémagglutinine.

**10.** Procédé selon la revendication 8, caractérisé en ce que ledit anticorps CD3 est G19-4/CD3.

**Revendications pour l'Etat contractant suivant : ES**

**1.** Procédé de fabrication d'une composition antivirale comprenant un conjugué de protéine antivirale pokeweed efficace pour inhiber la réplication d'un virus dans des cellules de mammifère infectées par un virus, sans cytotoxicité décelable, et un anticorps monoclonal réactif avec un antigène non associé au virus présent à la surface de ces cellules de mammifère, ce procédé comprenant la conjugaison de ladite protéine antivirale pokeweed avec ledit anticorps monoclonal humain, caractérisé en ce que ledit anticorps monoclonal est un anticorps monoclonal d'antigène CD4 ou CD5.

**2.** Procédé selon la revendication 1, caractérisé en ce que ledit anticorps est un anticorps monoclonal T101/CD5, 10.2/CD5 ou G17-2/CD4.

**3.** Procédé selon la revendication 2, caractérisé en ce que ledit anticorps monoclonal T101/CD5, 10.2/CD5 ou G17-2/CD4 et conjugué de protéine antivirale pokeweed est efficace pour inhiber la réplication de HIV ou de HTLV dans des cellules de mammifère infectées par HIV ou par HTLV.

**4.** Procédé pour inhiber la réplication de HIV dans des cellules de mammifère, caractérisé par le traitement d'une culture cellulaire comprenant des cellules T humaines infectées par HIV avec une quantité de la composition d'anticorps antiantigène CD4 humain et du conjugué de protéine antivirale pokeweed ou avec la composition d'anticorps antiantigène CD5 humain et du conjugué de protéine antivirale pokeweed, ou avec un mélange de celles-ci, efficace pour inhiber la réplication dudit HIV sans cytotoxicité décelable, ledit anticorps dudit conjugué étant réactif avec un antigène non associé au virus présent à la surface desdites cellules infectées par HIV.

**5.** Procédé selon la revendication 4, caractérisé en ce que ledit anticorps est un anticorps monoclonal G17-2/CD4 ou un autre anticorps où la liaison est essentiellement bloquée par G17-2 et qui bloque la liaison de G17-2/CD4.

**6.** Procédé selon la revendication 5, caractérisé en ce que ledit anticorps est un anticorps monoclonal 10.2/CD5 ou un autre anticorps où la liaison est essentiellement bloquée par 10.2 et qui bloque la liaison de 10.2/CD5.

**7.** Procédé pour inhiber la réplication de HIV dans des cellules de mammifère, caractérisé par
la stimulation des lymphocytes du sang périphérique obtenus à partir de patients infectés par HIV-1 avec une quantité d'un agent efficace pour provoquer une prolifération cellulaire et une réplication de HIV; et
le traitement desdits lymphocytes du sang périphérique avec une quantité d'une composition d'anticorps et de conjugué de protéine antivirale pokeweed efficace pour inhiber la réplication dudit HIV, dans lequel ledit anticorps est un anticorps monoclonal antiantigène CD4 ou anti-CD5 réactif avec un antigène non associé au virus présent à la surface de ces cellules de mammifère.

8. Procédé selon la revendication 7, caractérisé en ce que ledit agent de prolifération cellulaire et de réplication de HIV est un anticorps CD3.

9. Procédé selon la revendication 7, caractérisé en ce que ledit agent de prolifération cellulaire et de réplication de HIV est la phytohémagglutinine.

10. Procédé selon la revendication 8, caractérisé en ce que ledit anticorps CD3 est G19-4/CD3.

FIG. 1

MoAb−NH$_2$+ [N-hydroxy succinimide ester] N−O−C−(CH$_2$)$_2$−S−S−[2-pyridyl disulfide]

(N−hydroxy succinimide ester)  (2−pyridyl disulfide)

SPDP

MoAb−NH−C−(CH$_2$)$_2$−S−S−[pyridyl]

+ [N−OH]

PAP−NH$_2$ + [H$_2$C—CH$_2$ / H$_2$C S C−NH$_2$+ Cl]

2−Iminothiolano·HCl

PAP−NH−C−(CH$_2$)$_2$−SN
         ‖
         NH

         NH
         ‖
PAP−NH−C−(CH$_2$)$_2$−S−S−(CH$_2$)$_3$−C−NH−MoAb

Immunotoxin

+S−[pyridyl]

EP 0 441 917 B1

FIG. 2

EP 0 441 917 B1

FIG. 3A

FIG. 3B

## FIG. 4

FIG. 5

EP 0 441 917 B1

FIG. 6

FIG. 7

FIG. 8

FIG. 9

FIG. 10

FIG. 11A

FIG. 11B

FIG. 12A

FIG. 12B

FIG. 13

FIG14